(19) Europäisches Patentamt
European Patent Office
Office européen des brevets

(11) EP 1 824 852 B1

(12) **EUROPEAN PATENT SPECIFICATION**

(45) Date of publication and mention of the grant of the patent:
**14.05.2008 Bulletin 2008/20**

(21) Application number: **05811074.3**

(22) Date of filing: **30.11.2005**

(51) Int Cl.:
***C07D 471/14*** *(2006.01)* ***A61P 25/34*** *(2006.01)*

(86) International application number:
**PCT/IB2005/003730**

(87) International publication number:
**WO 2006/061711 (15.06.2006 Gazette 2006/24)**

(54) **1,2,3,3A,8,8A-HEXAHYDRO-2,7A-DIAZA-CYCLOPENTA[A]INDEN-7-ONE DERIVATIVES WHICH BIND TO NEURONAL NICOTINIC ACETYLCHOLINE SPECIFIC RECEPTOR SITES AND ARE USEFUL IN MODULATING CHOLINERGIC FUNCTION AND IN THE TREATMENT OF ADDICTIVE DISORDERS**

1,2,3,3A;8,8A-HEXAHYDRO-2,7A-DIAZACYCLOPENTA[A]INDEN-7-ONDERIVATE, DIE SICH AN NEURONALE NIKOTINISCHE ACETYLCHOLINSPEZIFISCHE REZEPTORSTELLEN BINDEN UND ZUR MODULATION DER CHOLINERGEN FUNKTION UND ZUR BEHANDLUNG VON SUCHTERKRANKUNGEN EIGNEN

DERIVES DE 1,2,3,3A,8,8A-HEXAHYDRO-2,7A-DIAZA-CYCLOPENTA[A]INDEN-7-ONE SE LIANT A DES SITES DE RECEPTEURS NICOTINIQUES NEURONAUX SPECIFIQUES DE L'ACETYLCHOLINE, UTILES DANS LA MODULATION DE LA FONCTION CHOLINERGIQUE ET DANS LE TRAITEMENT DE TROUBLES DE DEPENDANCE

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.12.2004 US 633898 P**

(43) Date of publication of application:
**29.08.2007 Bulletin 2007/35**

(73) Proprietor: **Pfizer Products Incorporated**
**Groton, CT 06340 (US)**

(72) Inventors:
- **O'NEILL, Brian, Thomas**
  **Eastern Point Road Groton, CT 06340 (US)**
- **PROCKO, Kristen**
  **Austin, TX 78712 (US)**
- **YOHANNES, Daniel**
  **New London, CT 06320 (US)**

(74) Representative: **Duncan, Garreth Andrew**
**Pfizer Limited**
**European Patents Department**
**Ramsgate Road**
**Sandwich, Kent CT13 9NJ (GB)**

(56) References cited:
**WO-A-98/18798** **WO-A-99/55680**

- **BOIDO C C ET AL: "SYNTHESIS AND PRLIMINARY PHARMACOLOGICAL EVALUATION OF SOME CYTISINE DERIVATIVES" FARMACO, SOCIETA CHIMICA ITALIANA, PAVIA, IT, vol. 54, no. 7, 30 July 1999 (1999-07-30), pages 438-451, XP001057032 ISSN: 0014-827X**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Background of the Invention

This invention relates to fused bicyclicpyrrolidine pyridone compounds, as defined more specifically by formula I below. Compounds of formula I bind to neuronal nicotinic acetylcholine specific receptor sites and are useful in modulating cholinergic function. Such compounds are useful in the treatment of addictive disorders such as the use of tobacco or other nicotine containing products and also for the treatment and prevention of withdrawal symptoms caused by cessation of chronic or long term use of tobacco products. These compounds are also useful in the treatment of neurological and mental disorders related to a decrease in cholinergic function such as Huntington's Chorea, tardive dyskinesia, hyperkinesia, mania, dyslexia, schizophrenia, analgesia, attention deficit disorder (ADD), multi-infarct dementia, age related cognitive decline, epilepsy, senile dementia of the Alzheimers type, Parkinson's disease, (PD) attention deficit hyperactivity disorder (ADHD), anxiety, obesity, Tourette's syndrome and ulcerative colitis.

The compounds of this invention may also be used in combination with an antidepressant such as a tricyclic antidepressant or a serotonin reuptake inhibiting antidepressant (SRI), in order to treat both the cognitive decline and depression associated with Alzheimers disease (AD), PD, stroke, Huntington's Chorea or traumatic brain injury (TBI); in combination with muscarinic agonists in order to stimulate both central muscarinic and nicotinic receptors for the treatment, for example, of ALS, cognitive dysfunction, age related cognitive decline, AD, PD, stroke, Huntington's Chorea and TBI; in combination with neurotrophic factors such as NGF in order to maximize cholinergic enhancement for the treatment, for example, of ALS, cognitive dysfunction, age related cognitive decline, AD, PD stroke, Huntington's Chorea and TBI; or in combination with agents that slow or arrest AD such as cognition enhancers, amyloid aggregation inhibitors, secretase inhibitors, tau kinase inhibitors, neuronal antiinflammatory agents and estrogen-like therapy.

Other compounds that bind to neuronal nicotinic receptor sites are referred to in United States Patent Application 08/963,852, which was filed on November 4, 1997, and in United States Provisional Patent Application 60/070,245, which was filed on December 31, 1997. Both of the foregoing applications are owned in common with the present application, and both are incorporated herein by reference in their entireties.

Summary of the Invention

This invention relates to fused bicyclicpyrrolidine pyridone compounds having the formula I:

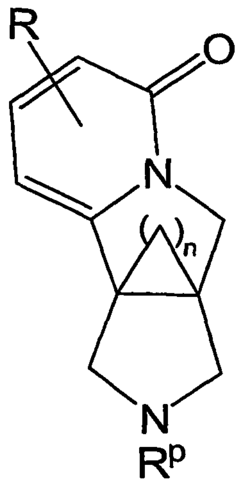

wherein $R^P$ is hydrogen, $(C_1-C_6)$alkyl, or benzyl;

R is hydrogen, $(C_1-C_6)$alkyl, $(C_2-C_6)$alkenyl, $(C_2-C_6)$alkynyl, hydroxy, amino, halo, cyano, aryl, wherein said aryl is selected from phenyl and naphthyl, heteroaryl, wherein said heteroaryl is selected from five to seven membered aromatic rings containing from one to four heteroatoms selected from oxygen, nitrogen and sulfur, $-SO_q(C_1-C_6)$alkyl or $-SO_q(C_1-C_6)$aryl wherein q is zero, one or two, $(C_1-C_6)$alkylamino-, $[(C_1-C_6)alkyl]_2$amino-, $-CO_2R^1$, $-CONR^2R^3$, $-SO_2NR^4R^5$, $-C(=O)R^6$, $-XC(=O)R^6$ wherein X is $(C_1-C_6)$alkylene, aryl-$(C_0-C_3)$alkyl- or aryl-$(C_0-C_3)$alkyl-O-, heteroaryl-$(C_0-C_3)$alkyl- or heteroaryl-$(C_0-C_3)$alky]-O-, and $X^2(C_0-C_6)$alkoxy-$(C_0-C_6)$alkyl-, wherein $X^2$ is absent or $X^2$ is $(C_1-C_6)$alkylamino- or $[(C_1-C_6)alkyl]_2$amino-, and wherein the $(C_0-C_6)$alkoxy-$(C_0-C_6)$alkyl- moiety of said $X^2(C_0-C_6)$alkoxy-$(C_0-C_6)$alkyl- contains at least one carbon atom, and wherein from one to three of the carbon atoms of said $(C_0-C_6)$alkoxy-$(C_0-C_6)$alkyl-

moiety may optionally be replaced by an oxygen, nitrogen or sulfur atom, with the proviso that any two such heteroatoms must be separated by at least two carbon atoms, and wherein any of the alkyl moieties of said $(C_0-C_6)$alkoxy-$(C_0-C_6)$alkyl- may be optionally substituted with from two to seven fluorine atoms, and wherein one of the carbon atoms of each of the alkyl moieties of said aryl-$(C_0-C_3)$alkyl- and said heteroaryl-$(C_0-C_3)$alkyl- may optionally be replaced by an oxygen, nitrogen or sulfur atom, and wherein each of the foregoing aryl and heteroaryl groups may optionally be substituted with one or more substituents, preferably from zero to two substituents, independently selected from $(C_1-C_6)$alkyl optionally substituted with from one to seven fluorine atoms, $(C_1-C_6)$alkoxy optionally substituted with from two to seven fluorine atoms, halo selected from chloro, fluoro, bromo and iodo, $(C_2-C_6)$alkenyl, $(C_2-C_6)$alkynyl, hydroxy, nitro, cyano, amino, $(C_1-C_6)$alkylamino-, $[(C_1-C_6)$ alkyl$]_2$amino-, $-CO_2R^1$, $-CONR^2R^3$, $-SO_2NR^4R^5$, $-C(=O)R^6$ and $-XC(=O)R^6$;

each $R^1$, $R^2$, $R^3$, $R^4$, $R^5$ and $R^6$ is selected, independently, from hydrogen and $(C_1 - C_6)$ alkyl, or $R^2$ and $R^3$, or $R^4$ and $R^5$ together with the nitrogen to which they are attached, form a pyrrolidine, piperidine, morpholine, azetidine, piperizine, -N-$(C_1-C_6)$alkylpiperizine or thiomorpholine ring, or a thiomorpholine ring wherein the ring sulfur is replaced with a sulfoxide or sulfone;

each X is, independently, $(C_1-C_6)$alkylene; and,

n is an integer from zero to 2; or, a pharmaceutically acceptable salt of such a compound.

In another aspect, the present invention also relates to a compound of the formula I wherein $R^P$ is a protective group selected from t-butoxycarbonyl (t-Boc), trifluoroacetyl, CBz, FMOC, Bz, methyl and acetyl.

Preferred embodiments include the compound wherein R is hydrogen, $(C_1-C_6)$alkyl, $(C_1-C_6)$alkoxy, $CF_3O$, acetyl, $C(=O)R^6$, halo, cyano, phenyl, and heteroaryl, where said heteroaryl is pyridyl, oxazolyl, isooxazolyl, thiazolyl or isothiazolyl.

Unless otherwise indicated, the term "halo", as used herein, includes fluoro, chloro, bromo and iodo.

Unless otherwise indicated, the term "alkyl", as used herein, includes straight, branched or cyclic, and may include straight and cyclic alkyl moieties as well as branched and cyclic moieties.

The term "alkoxy", as used herein, means "alkyl-O-", wherein "alkyl" is defined as above.

The term "alkylene, as used herein, means an alkyl radical having two available bonding sites (i.e., -alkyl-), wherein "alkyl" is defined as above.

The term "alkenyl" is intended to include hydrocarbon chains of either a straight or branched configuration comprising one or more unsaturated carbon-carbon bonds which may occur in any stable point along the chain, such as ethenyl and propenyl. Alkenyl groups typically will have 2 to about 12 carbon atoms, more typically 2 to about 8 carbon atoms.

The term "alkynyl" is intended to include hydrocarbon chains of either a straight or branched configuration comprising one or more triple carbon-carbon bonds which may occur in any stable point along the chain, such as ethynyl and propynyl. Alkynyl groups typically will have 2 to about 12 carbon atoms, more typically 2 to about 8 carbon atoms.

The term "aryl is intended to include groups that, in accordance with the theory of Hückel, have a cyclic, delocalized (4n +2) pi-electron system. Examples of aryl groups include, but are not limited to, arenes and their substitution products, e.g. phenyl, naphthyl and toluyl, among numerous others.

The term "heteroaryl" is intended to include aromatic heterocyclic groups and includes the non-limiting examples thiophenyl, pyridyl, pyrimidyl, pyridazyl, oxazolyl, isooxazolyl, thiazolyl and isothiazolyl, among others.

Unless otherwise indicated, the term "one or more substituents", as used herein, refers to from one to the maximum number of substituents possible based on the number of available bonding sites.

The compounds of formula I may have chiral centers and therefore may occur in different enantiomeric configurations. The invention includes all enantiomers, structurally allowable diastereomers, and other stereoisomers of such compounds of formula I, as well as racemic and other mixtures thereof.

The present invention also relates to a pharmaceutical composition for use in reducing nicotine addiction or aiding in the cessation or lessening of tobacco use in a mammal, including a human, comprising an amount of a compound of the formula 1, or a pharmaceutically acceptable salt thereof, that is effective in reducing nicotine addiction or aiding in the cessation or lessening of tobacco use and a pharmaceutically acceptable carrier.

The present invention also relates to a method for reducing nicotine addiction or aiding in the cessation or lessening of tobacco use in a mammal, including a human, comprising administering to said mammal an amount of a compound of the formula I, or a pharmaceutically acceptable salt thereof, that is effective in reducing nicotine addiction or aiding in the cessation or lessening of tobacco use.

The present invention also relates to a method of treating a disorder or condition selected from inflammatory bowel disease (including but not limited to ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amylotropic lateral sclerosis (ALS), cognitive dysfunction, hypertension, bulimia, anorexia, obesity, cardiac arrythmias, gastric acid hypersecretion, ulcers, pheochromocytoma, progressive supramuscular palsy, chemical dependencies and addictions (e.g., dependencies on, or addictions to nicotine (and/or tobacco products), alcohol, benzodiazepines, barbituates, opioids or cocaine), headache, stroke,

traumatic brain injury (TBI), psychosis, Huntington's Chorea, tardive dyskinesia, hyperkinesia, dyslexia, schizophrenia, multi-infarct dementia, age related cognitive decline, epilepsy, including petit mal absence epilepsy, senile dementia of the Alzheimer's type (AD), Parkinson's disease (PD), attention deficit hyperactivity disorder (ADHD) and Tourette's Syndrome in a mammal, comprising administering to a mammal in need of such treatment an amount of a compound of the formula I, or a pharmaceutically acceptable salt thereof, that is effective in treating such disorder or condition.

The present invention also relates to a pharmaceutical composition for treating a disorder or condition selected from inflammatory bowel disease (including but not limited to ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amylotropic lateral sclerosis (ALS), cognitive dysfunction, hypertension, bulimia, anorexia, obesity, cardiac arrythmias, gastric acid hypersecretion, ulcers, pheochromocytoma, progressive supramuscular palsy, chemical dependencies and addictions (e.g., dependencies on, or addictions to nicotine (and/or tobacco products), alcohol, benzodiazepines, barbituates, opioids or cocaine), headache, stroke, traumatic brain injury (TBI), psychosis, Huntington's Chorea, tardive dyskinesia, hyperkinesia, dyslexia, schizophrenia, multi-infarct dementia, age related cognitive decline, epilepsy, including petit mal absence epilepsy, senile dementia of the Alzheimer's type (AD), Parkinson's disease (PD), attention deficit hyperactivity disorder (ADHD) and Tourette's Syndrome in a mammal, comprising an amount of a compound of the formula I, or a pharmaceutically accepable salt thereof, and a pharmaceutically acceptable carrier.

This invention also relates to the pharmaceutically acceptable acid addition salts of the compounds of formula I. Examples of pharmaceutically acceptable acid addition salts of the compounds of formula I are the salts of hydrochloric acid, p-toluenesulfonic acid, fumaric acid, citric acid, succinic acid, lactic acid, acetic acid, maleic acid, salicylic acid, oxalic acid, hydrobromic acid, phosphoric acid, methanesulfonic acid, tartaric acid, malate, di-p-toluoyl tartaric acid, and mandelic acid.

Detailed Description of the Invention

The compounds of formula I can be prepared according to the methods of Schemes 1 - 4. Except where otherwise stated, R and $R^1$ through $R^6$ in the reaction schemes and discussion that follow are defined as above. Unless otherwise stated reaction conditions include an inert atmosphere commonly used in the art such as nitrogen or argon.

SCHEME 1

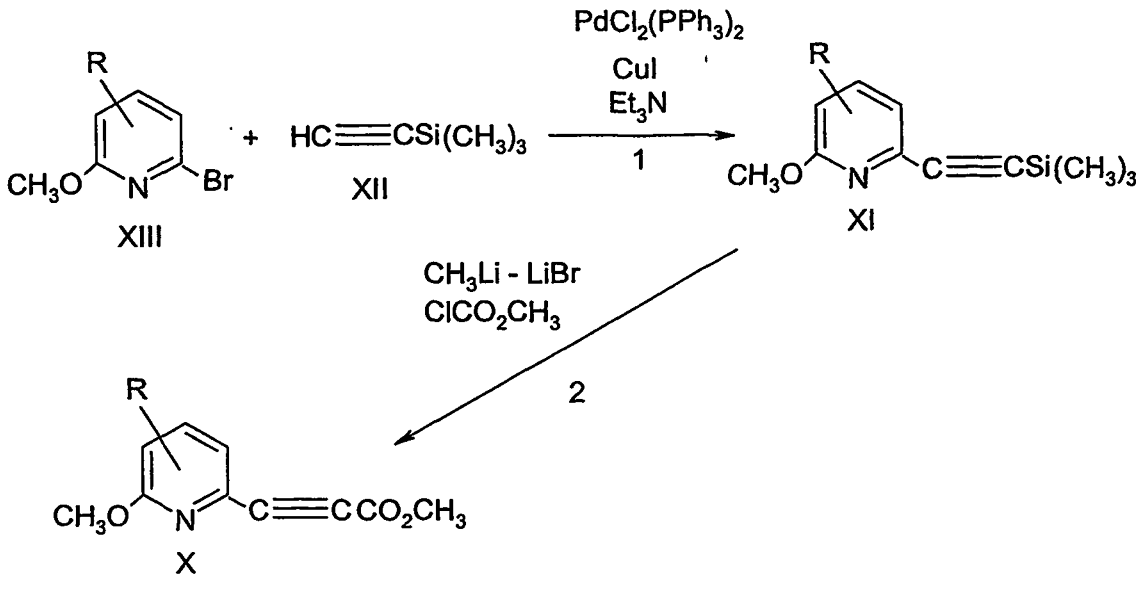

SCHEME 2

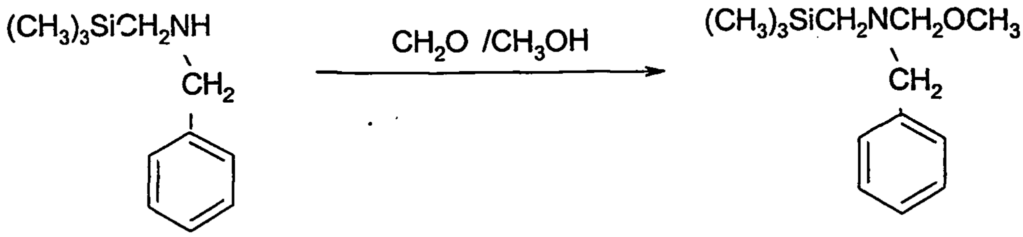


IX

VIII

SCHEME 3

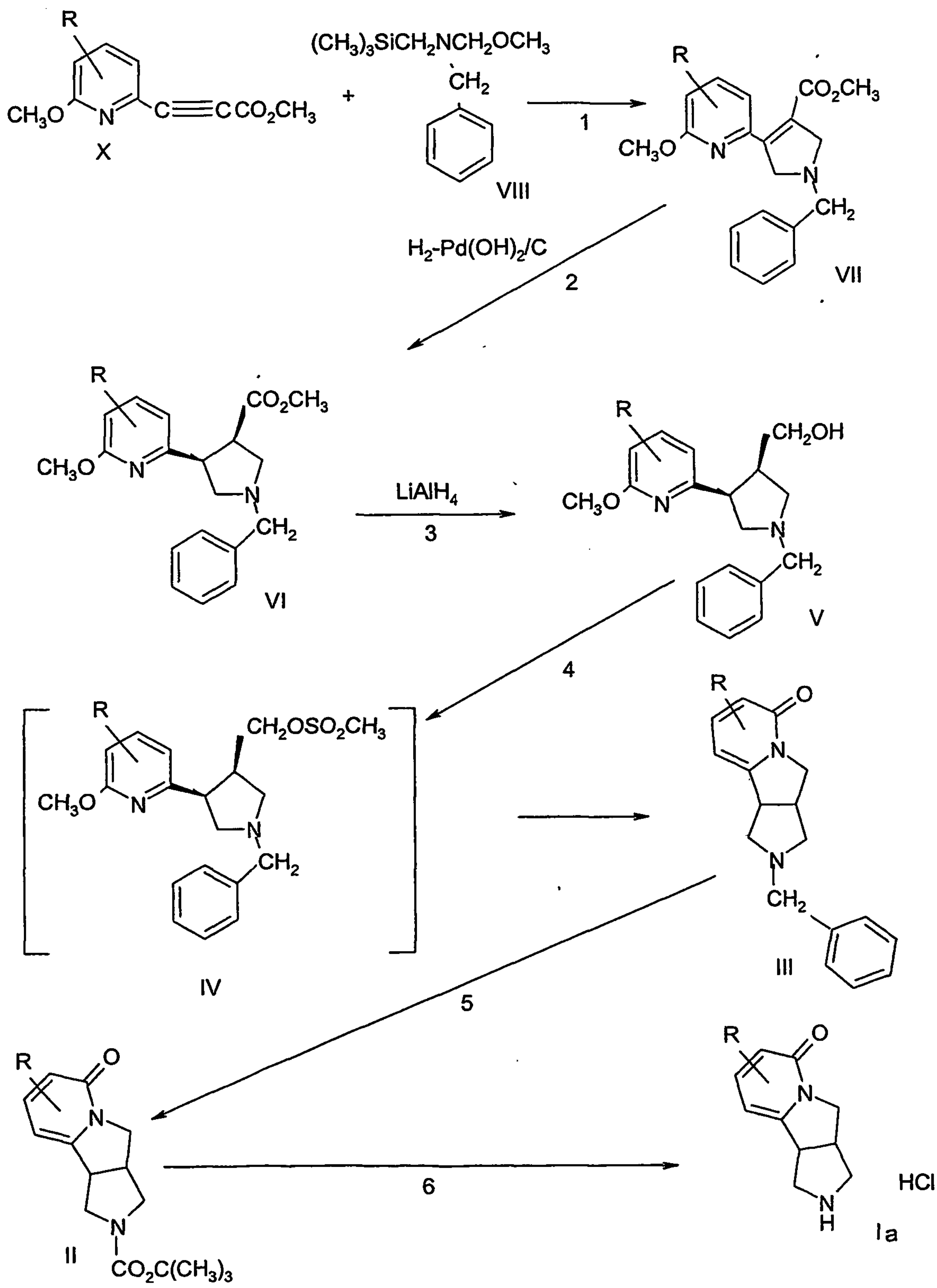

SCHEME 4

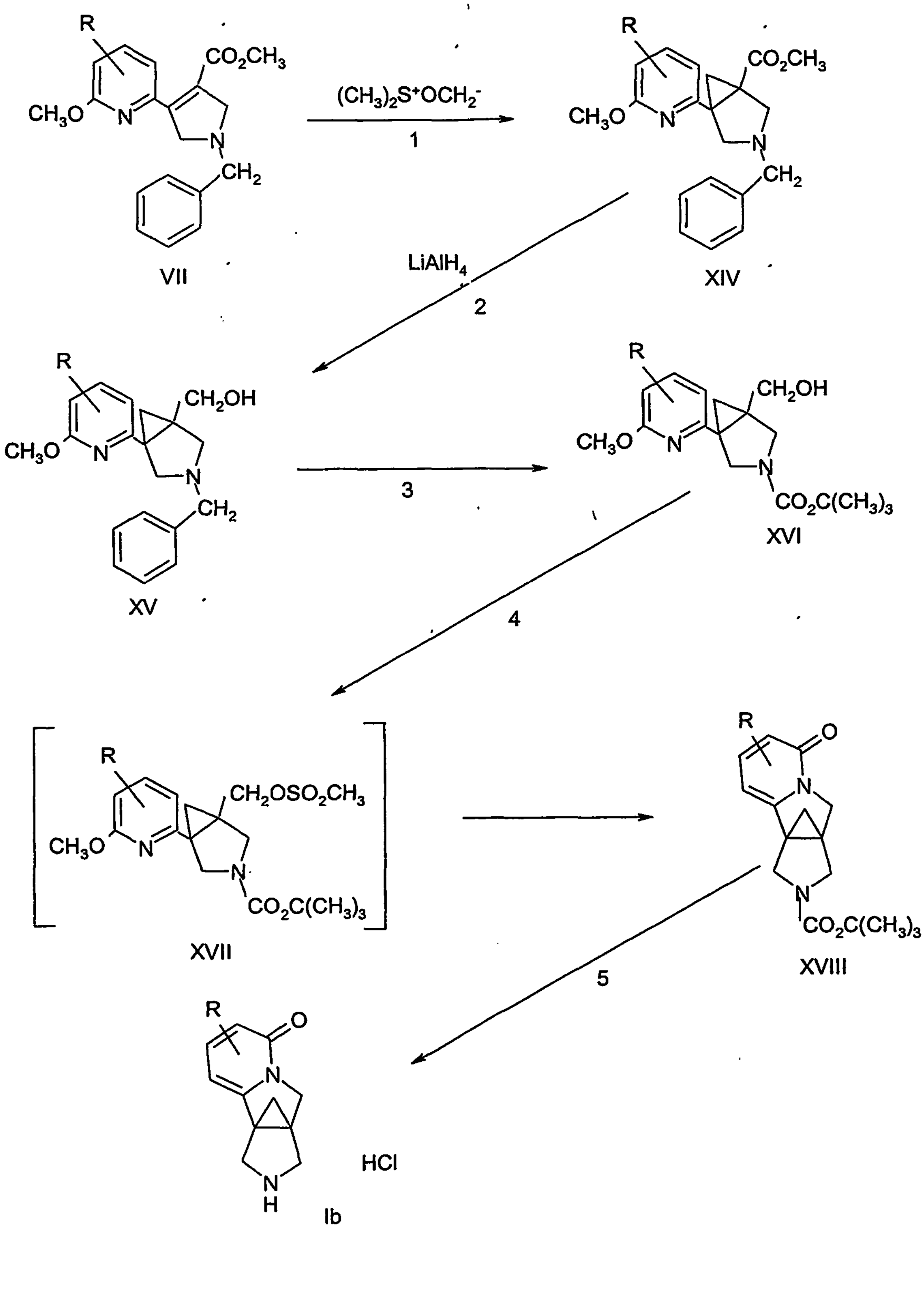

SCHEME 5

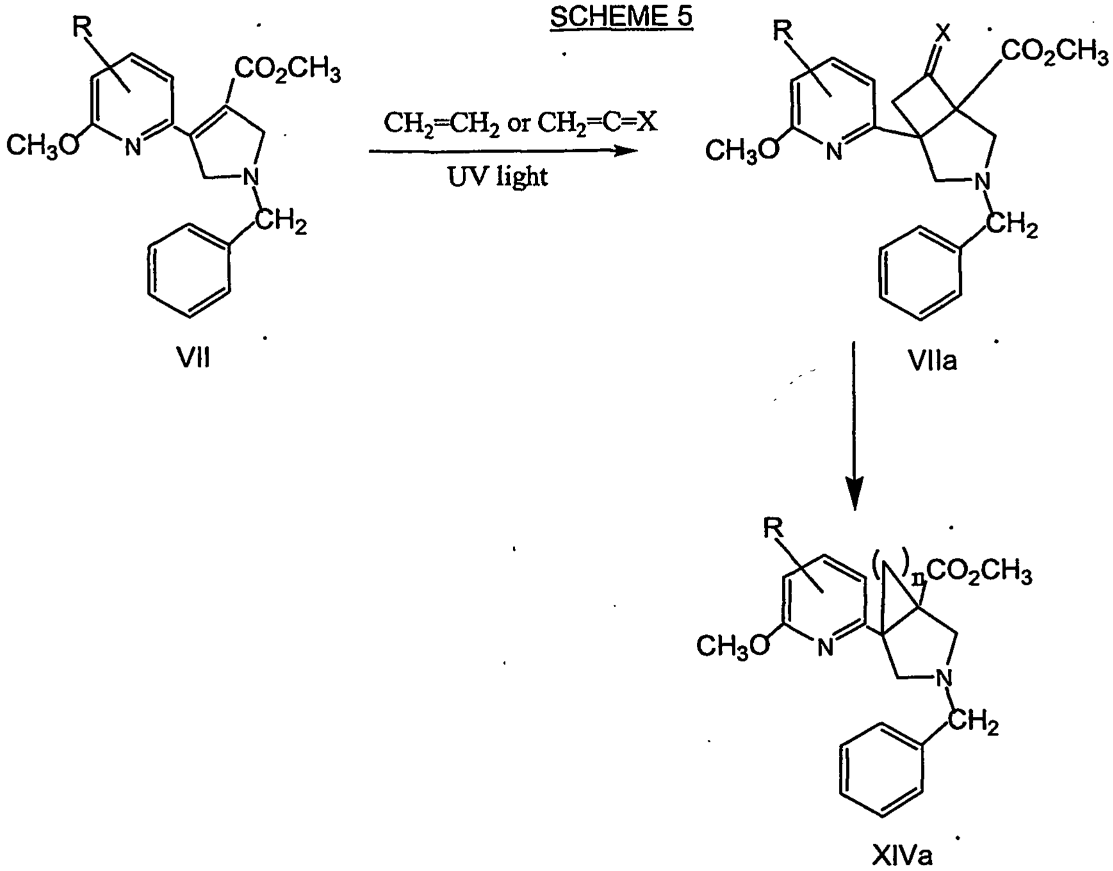


Scheme 1 refers to the preparation of precursor compound X, used in the preparation of compounds of the formula I or Ia, from compounds of the formulas XII and XIII. Compounds XII and XIII are commercially available or can be prepared by methods well known to those of ordinary skill in the art.

In step 1 of scheme 1, a compound of the formula XI is prepared by replacing the halo group of a compound of the formula XIII with a trialkylsilanylethynyl group by treating the compound of formula XIII with a (trialkylsilyl)acetylene, preferably (trimethylsilyl)acetylene, formula XII, in a tertiary alkyl amine solvent, preferably triethylamine, with catalytic amounts of a mixture of $PdCl_2(Ph_3P)_2$ and CuI halide at about 80°C to about 90°C preferably at about the reflux temperature of triethylamine, for about 1.5 hours to about 3.5 hours, preferably about 2.5 hours. The process may be carried out with a compound bearing I instead of Br. Other catalysts which can be used include $PdCl_2$, $Pd(OAc)_2$, $Pd(dppf)_2Cl_2$, $Pd(dppe)_2Cl_2$, Pd/C, and $Pd(PPh_3)_4$.

In step 2 of Scheme 1 a compound of the formula X is prepared by treating a compound of the formula XI with an alkyllithium complexed with a lithium halide, preferably methyllithium complexed with lithium bromide in a reaction inert solvent preferably an ethereal solvent such as diethyl ether, dioxane or tetrahydrofuran (THF), most preferably THF, at a temperature of about -80°C to about 0°C, preferably by slowly adding the alkyllithium lithium halide complex at about -78°C and then warming to about 0°C for about 10 minutes to about 3 hours. The reaction mixture is then cooled to about -10°C to about -30°C preferably about - 20°C and quenched with an approximately equivalent amount of alkyl haloformate, preferably methyl chloroformate and then allowed to warm to room temperature.

Scheme 2 refers to the preparation of precursor compound VIII, used in the preparation of compounds of the formula I or Ia, from a compound of the formula IX. Compound IX is commercially available or can be prepared by methods well known to those of ordinary skill in the art. A compound of the formula VIII can be prepared by treating a compound of the formula IX with formaldehyde at a temperature of about -10°C to about 10°C preferably about 0°C for about 5 to about 25 minutes, preferably about 10 minutes and then treating with methanol followed by an excess of alkaline metal carbonate, preferably potassium carbonate, stirring for about 0.5 to about 2 hours, preferably about 1 hour and then separating the liquid phase. Additional alkaline metal carbonate, preferably potassium carbonate, may be added to the liquid phase and the reaction mixture stirred at room temperature for up to about 72 hours to ensure completeness of reaction.

Scheme 3 refers to the preparation of a compound of the formula I wherein n = 0. In step 1 of Scheme 3, a

compound of the formula VII, which is a precursor of the compound I, is prepared from a compound of the formula X, and a compound of the formula VIII by mixing the compound of the formula X with the compound of the formula VIII in a chlorinated hydrocarbon solvent such as chloroform, dichoroethane (DCE) or methylene chloride, preferably methylene chloride. The stirred mixture is cooled to about -5°C to about 5°C, preferably about 0°C, and is then treated with trifluoroacetic acid (TFA) in a chlorinated hydrocarbon solvent such as chloroform, dichoroethane (DCE) or methylene chloride, preferably methylene chloride and maintained at about -5°C to about 5°C, preferably about 0°C, for about 10 minutes to about 30 minutes, preferably about 20 minutes. The mixture is then allowed to warm to room temperature and stirred for about 1 hour to about 3 hours, preferably about 2 hours.

In step 2 of Scheme 3, a compound of the formula VI is prepared by reducing the double bond of the compound of formula VII, preferably by catalytic hydrogenation using standard techniques that are well known to those skilled in the art. For example, reduction of the double bond may be effected with hydrogen gas ($H_2$) using catalysts such as palladium on carbon (Pd/C), palladium on barium sulfate ($Pd/BaSO_4$), palladium hydroxide, platinum on carbon (Pt/C), or tris(triphenylphosphine)rhodium chloride (Wilkinson's catalyst), in an appropriate solvent such as methanol, ethanol, THF, dioxane or ethyl acetate, at a pressure from about 1 to about 5 atmospheres and a temperature from about 10°C to about 60°C, as described in Catalytic Hydrogenation in Organic Synthesis, Paul Rylander, Academic Press Inc., San Diego, 1979, pp 31-63. The following conditions are preferred: hydrogenation in methanol in the presence of a palladium hydroxide/activated carbon catalyst at about 30 psi to about 50 psi, preferably about 40 psi and about 20°C to about 25°C for about 1.5 hours to about 2.5 hours, preferably about 2 hours.

In step 3 of Scheme 3, a compound of the formula V is prepared by reducing the ester group of the compound of formula VI to a methylene hydroxy group with a hydride reducing agent, preferably lithium aluminum hydride, in a suitable solvent such as diethyl ether, dioxane or THF, preferably diethyl ether, at about -10°C to about 10°C, preferably about 0°C, for about 1 hour to about 2 hours, preferably about 1.5 hours. Other possible hydride reducing agents include lithium borohydride, Dibal-H™, Red-Al™, and Vitride™.

In step 4 of Scheme 3, a compound of the formula III is prepared by treating the compound of the formula V with a tertiary amine, preferably a hindered tertiary alkyl amine and an alkyl or aryl sulfonyl halide in a dry reaction inert solvent to form an intermediate sulfonic ester exemplified by the compound of formula IV, which then cyclizes to the compound of formula III. Suitable solvents include chloroform, dichoroethane (DCE) or methylene chloride, or other chlorinated hydrocarbon solvents, preferably methylene chloride. Suitable tertiary amines include triethylamine, tributylamine, dimethylaminopyridine and N,N diisopropylethylamine, preferably a hindered tertiary alkyl amine such as N, N diisopropylethylamine. Suitable sulfonylhalides include toluenesulfonyl chloride, benzenesulfonyl chloride and methylsulfonyl chloride, preferably methylsulfonyl chloride. The temperature of the aforesaid reaction wherein a sulfonylhalide is added to a mixture of the compound of formula V, tertiary amine and solvent, is in the range from about -10°C to about 10°C, preferably about 0°C, for about 30 minutes to about 2 hours, preferably about 1 hour. The reaction mixture is then allowed to warm to about 15°C to about 30°C, preferably about 20°C to about 25°C, and stirred for about 3 hours to about 24 hours, preferably about 16 hours.

In step 5 of Scheme 3, a compound of the formula II is prepared by catalytic hydrogenation of the compound of formula III in the presence of di-tertiary-butyl dicarbonate (Boc anhydride) in order to remove the protective benzyl group and replace it with a t-BOC group. Suitable catalysts, solvents and reaction conditions for the removal of protective benzyl groups by catalytic hydrogenation are well known to those skilled in the art. Preferred conditions are as follows: palladium hydroxide on activated carbon, methanol solvent at about 30 psi to about 60 psi, more preferably about 45 psi and about 25°C to about 75°C, more preferably 50°C, for about 30 minutes to about 2.5 hours, more preferably about 1 hour.

In step 6 of Scheme 3, a solution of the compound of formula II is treated with acid in a solvent, according to procedures known in the art for removal of a Boc protective group, to form a compound of formula I wherein n = 0. Suitable acids include strong organic or mineral acids, preferably HCl. Suitable solvents include lower alkyl alcoholic solvents such as, methanol, ethanol, 1 or 2 propanol, esters such as methyl, ethyl or butyl acetate, or ethers such as THF, dioxane or mixtures thereof, preferably a mixture of methanol and ethyl acetate for the compound of formula II and methanol for HCl. The temperature of the aforesaid reaction is from about 15°C to about 30°C, preferably about 20°C to about 25°C for about 3 hours to about 18 hours, preferably about 16 hours. To ensure completeness of reaction an additional amount of acid, preferably HCl, is added at the end of this time and the reaction mixture is heated to about 40°C to about 60°C, preferably 50°C for about 2 hours to about 6 hours, preferably about 4 hours. After standard procedures the compound of formula I is isolated as a salt, preferably the hydrochloride salt.

Scheme 4 refers to the preparation of a compound of the formula I wherein n = 1, designated 1a in Scheme 4. In step 1 of Scheme 4, a compound of the formula XIV is prepared by treating a compound of the formula VII with a reactive methylene compound in a suitable solvent to convert the double bond of compound VII to a cyclopropane bridge. Suitable reactive methylene compounds include ylides such as triphenylphosphonium ylide, dimethylsulfoxonium ylide and diazomethane, preferably dimethylsulfoxonium ylide prepared using standard techniques that are well known to those skilled in the art. Suitable solvents include THF, diethyl ether and dioxane, preferably THF. The temperature of

the aforesaid reaction is about 15°C to about 30°C, preferably about 20°C to about 25°C, for about 1 hour to about 2 hours, preferably about 1.5 hours.

In step 2 of Scheme 4, a compound of the formula XV is prepared by reducing the ester group of the compound of formula XIV to a methylene hydroxy group with a hydride reducing agent, preferably lithium aluminum hydride, in a suitable solvent such as diethyl ether, dioxane or THF, preferably diethyl ether, at about -10°C to about 10°C, preferably about 0°C, for about 1 hour to about 2 hours, preferably about 1.5 hours. Other reducing agents useful for the reaction include lithium borohydride, Dibal-H™, Red-Al™, and Vitride™.

In step 3 of Scheme 4, a compound of the formula XVI is prepared by catalytic hydrogenation of the compound of formula XV in the presence of di-tertiary-butyl dicarbonate (Boc anhydride) in order to remove the protective benzyl group and replace it with a t-BOC group. Suitable catalysts, solvents and reaction conditions for the removal of protective benzyl groups by catalytic hydrogenation are well known to those skilled in the art. Preferred conditions are as follows: palladium hydroxide on activated carbon, methanol solvent at about 30 psi to about 60 psi, more preferably about 45 psi and about 25°C to about 75°C, more preferably 50°C, for about 30 minutes to about 2.5 hours, more preferably about 1 hour.

In step 4 of Scheme 4, a compound of the formula XVIII is prepared by treating the compound of the formula XVI with a tertiary amine, preferably a hindered tertiary alkyl amine and an alkyl or aryl sulfonyl halide in a dry reaction inert solvent to form an intermediate sulfonic ester exemplified by the compound of formula XVII, which then cyclizes to the compound of formula XVIII. Suitable solvents include chloroform, dichoroethane (DCE) or methylene chloride, or other chlorinated hydrocarbon solvents, preferably methylene chloride. Suitable tertiary amines include triethylamine, tributylamine, dimethylaminopyridine and N,N diisopropylethylamine, preferably a hindered tertiary alkyl amine such as N,N diisopropylethylamine. Suitable sulfonylhalides include toluenesulfonyl chloride, benzenesulfonyl chloride and methylsulfonyl chloride, preferably methylsulfonyl chloride. The temperature of the aforesaid reaction wherein a sulfonylhalide is added to a mixture of the compound of formula V, tertiary amine and solvent, is in the range from about -10°C to about 10°C, preferably about 0°C, for about 30 minutes to about 2 hours, preferably about 1 hour. The reaction mixture is then allowed to warm to about 15°C to about 30°C, preferably about 20°C to about 25°C and stirred for about 3 hours to about 24 hours, preferably about 16 hours.

In step 5 of Scheme 4, a solution of the compound of formula XVIII is treated with acid in a solvent, according to procedures known in the art for removal of a Boc protective group, to form a compound of formula Ib (compound I wherein n = 1). Suitable acids include strong organic or mineral acids, preferably HCl. Suitable solvents include lower alkyl alcoholic solvents such as, methanol, ethanol, 1 or 2 propanol, esters such as methyl, ethyl or butyl acetate, or ethers such as THF, dioxane, lower alkyl hydrocarbon solvents such as hexanes or mixtures thereof, preferably a mixture of hexanes and ethyl acetate for the compound of formula XVIII and methanol for HCl. The temperature of the aforesaid reaction is from about 15°C to about 30°C, preferably about 20°C to about 25°C for about 3 hours to about 18 hours, preferably about 16 hours. To ensure completeness of reaction an additional amount of acid, preferably HCl, is added at the end of this time and the reaction mixture is heated to about 40°C to about 60°C, preferably 50°C for about 2 hours to about 6 hours, preferably about 4 hours. After standard procedures the compound of formula Ib is isolated as a salt, preferably the hydrochloride salt.

Scheme 5 shows how compounds of the invention wherein n=2 are prepared. Reaction conditions are readily determined based on similar [2 + 2] photocycloadditions known in the art. Lo, P. et al., Org. Lett., 3, 2819 (2001); Crimmins, M.T., et al., Tetrahedron Lett., 35, 1657-60 (1994); Herzog. H., et al., Tetrahedron, 42, 3547-58 (1986); Tobe, Y., et al., Tetrahedron Left., 27, 2905-06 (1986).

Compounds of formula I wherein $R^P$ is $(C_1-C_6)$alkyl or benzyl may be produced by treating compounds of formula Ia and Ib with $(C_1-C_6)$alkyl halides and benzyl halides by methods well known in the art.

In each of the reactions discussed above, or illustrated in Schemes 1-4, above, pressure is not critical unless otherwise indicated. Pressures from about 0.5 atmospheres to about 5 atmospheres are generally acceptable, with ambient pressure, i.e., about 1 atmosphere, being preferred as a matter of convenience.

The compounds of the formula I and their pharmaceutically acceptable salts (hereafter "the active compounds") can be administered via either the oral, transdermal (e.g., through the use of a patch), intranasal, sublingual, rectal, parenteral or topical routes. Transdermal and oral administration are preferred. These compounds are, most desirably, administered in dosages ranging from about 0.25 mg up to about 1500 mg per day, preferably from about 0.25 to about 300 mg per day in single or divided doses, although variations will necessarily occur depending upon the weight and condition of the subject being treated and the particular route of administration chosen. However, a dosage level that is in the range of about 0.01 mg to about 10 mg per kg of body weight per day is most desirably employed. Variations may nevertheless occur depending upon the weight and condition of the persons being treated and their individual responses to said medicament, as well as on the type of pharmaceutical formulation chosen and the time period and interval during which such administration is carried out. In some instances, dosage levels below the lower limit of the aforesaid range may be more than adequate, while in other cases still larger doses may be employed without causing any harmful side effects, provided that such larger doses are first divided into several small doses for administration throughout the day.

The active compounds can be administered alone or in combination with pharmaceutically acceptable carriers or diluents by any of the several routes previously indicated. More particularly, the active compounds can be administered in a wide variety of different dosage forms, e.g., they may be combined with various pharmaceutically acceptable inert carriers in the form of tablets, capsules, transdermal patches, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents. In addition, oral pharmaceutical compositions can be suitably sweetened and/or flavored. In general, the active compounds are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc can be used for tabletting purposes. Solid compositions of a similar type may also be employed as fillers in gelatin capsules; preferred materials in this connection also include lactose or milk sugar] as well as high molecular weight polyethylene glycols. When aqueous suspensions and/or elixirs are desired for oral administration the active ingredient may be combined with various sweetening or flavoring agents, coloring matter and, if so desired, emulsifying and/or suspending agents, together with such diluents as water, ethanol, propylene glycol, glycerin and various combinations thereof.

For parenteral administration, a solution of an active compound in either sesame or peanut oil or in aqueous propylene glycol can be employed. The aqueous solutions should be suitably buffered (preferably pH greater than 8), if necessary, and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

It is also possible to administer the active compounds topically and this can be done by way of creams, a patch, jellies, gels, pastes, ointments and the like, in accordance with standard pharmaceutical practice.

Biological Assay

The effectiveness of the active compounds in suppressing nicotine binding to specific receptor sites is determined by the following procedure which is a modification of the methods of Lippiello, P. M. and Fernandes, K. G. (in The Binding of L-[3H]Nicotine To A Single Class of High-Affinity Sites in Rat Brain Membranes, Molecular Pharm., 29; 448-54, (1986)) and Anderson, D. J. and Arneric, S. P. (in Nicotinic Receptor Binding of 3H-Cystisine, 3H-Nicotine and 3H-Methylcarmbamylcholine In Rat Brain, European J. Pharm., 253, 261-67 (1994)).

Procedure

Male Sprague-Dawley rats (200-300 g) from Charles River were housed in groups in hanging stainless steel wire cages and were maintained on a 12 hour light/dark cycle (7 a.m.-7 p.m. light period). They received standard Purina Rat Chow and water *ad libitum.*

The rats were killed by decapitation. Brains were removed immediately following decapitation. Membranes were prepared from brain tissue according to the methods of Lippiello and Fernandez (Molec Pharmacol, 29, 448-454, (1986) with some modification. Whole brains were removed, rinsed with ice-cold buffer, and homogenized at 0° in 10 volumes of buffer (w/v) using a Brinkmann Polytron™, setting 6, for 30 seconds. The buffer consisted of 50 mM Tris HCl at a pH of 7.5 at room temperature. The homogenate was sedimented by centrifugation (10 minutes; 50,000 x g; 0 to 4°C. The supernatant was poured off and the membranes were gently resuspended with the Polytron and centrifuged again (10 minutes; 50,000 x g; 0 to 4°C. After the second centrifugation, the membranes were resuspended in assay buffer at a concentration of 1.0g/100mL. The composition of the standard assay buffer was 50 mM Tris HCl, 120 mM NaCl, 5 mM KCl, 2 mM $MgCl_2$, 2 mM $CaCl_2$ and has a pH of 7.4 at room temperature.

Routine assays were performed in borosilicate glass test tubes. The assay mixture typically consisted of 0.9 mg of membrane protein in a final incubation volume of 1.0 mL. Three sets of tubes were prepared wherein the tubes in each set contained 50μL of vehicle, blank, or test compound solution, respectively. To each tube was added 200 μL of [$^3$H]-nicotine in assay buffer followed by 750 μL of the membrane suspension. The final concentration of nicotine in each tube was 0.9 nM. The final concentration of cytisine in the blank was 1 μM. The vehicle consisted of deionized water containing 30 μL of 1 N acetic acid per 50 mL of water. The test compounds and cytisine were dissolved in vehicle. Assays were initiated by vortexing after addition of the membrane suspension to the tube. The samples were incubated at 0 to 4° C in an iced shaking water bath. Incubations were terminated by rapid filtration under vacuum through Whatman GF/B™ glass fiber filters using a Brandel™ multi-manifold tissue harvester. Following the initial filtration of the assay

mixture, filters were washed two times with ice-cold assay buffer (5 m each). The filters were then placed in counting vials and mixed vigorously with 20 ml of Ready Safe™ (Beckman) before quantification of radioactivity. Samples were counted in a LKB Wallach Rackbeta™ liquid scintillation counter at 40-50% efficiency. All determinations were in triplicate.

Calculations

Specific binding (C) to the membrane is the difference between total binding in the samples containing vehicle only and membrane (A) and non-specific binding in the samples containing the membrane and cytisine (B), i.e.,

$$\text{Specific binding} = (C) = (A) - (B).$$

Specific binding in the presence of the test compound (E) is the difference between the total binding in the presence of the test compound (D) and non-specific binding (B), i.e., (E) = (D) -(B).

$$\text{\% Inhibition} = (1-((E)/(C)) \text{ times } 100.$$

The compounds of the invention that were tested in the above assay exhibited $IC_{50}$ values of less than 9 μM, and greater than 1 μM.
The following experimental examples illustrate, but do not limit the scope of, this invention.

**EXAMPLE 1**

**1,2,3,3a,8,8a-Hexahydro-2,7a-diaza-cyclopenta[a]inden-7-one (hydrochloride salt)**

A) 2-Methoxy-6-trimethylsilanylethynyl-pyridine

To a 300 mL three-necked round-bottom flask equipped with a condenser and nitrogen inlet was added 5g (26.6 mmol) of 2-bromo-6-methoxypyridine, 4.2g (42.9 mmol) of (trimethylsilyl)acetylene, and 120 mL of triethylamine. The above solution was treated with 479 mg (0.685 mmol) $PdCl_2(Ph_3P)_2$, followed by the addition of 235 mg (1.26 mmol) CuI. The reaction mixture was then heated under reflux for 2.5 hours, then allowed to cool to room temperature. The reaction mixture was filtered through diatomaceous earth and the solvent evaporated. Flash 40 chromatography (40-L) eluting with 20% methylene chloride/hexanes afforded 4.42 g of the product as a yellow oil, 81%. Mass spectrum (APCI) m/e 206 p+1. $^1H$ NMR ($CDCl_3$, 400 MHz)_7.47(dd, J = 7.1 Hz, 7.5 Hz, J = 18.5 Hz, 1H), 7.05 (d, J = 7.1 Hz, 1H), 6.65 (d, J = 7.5 Hz, 1H), 3.91 (s, 3H), 0.235 (s, 9H) ppm.

B) 3-(6-Methoxy-pyridin-2-yl)-prop-2-ynoic acid methyl ester

To a 1000 mL round bottom flask under nitrogen was added 30.1g (151 mmol) of 2-Methoxy-6-trimethylsilanylethynyl-pyridine and 450 mL of THF. The reaction mixture was cooled to -78°C and treated with drop-wise addition of 105 mL (158 mmol) of methyllithium complexed with lithium bromide, 1.5 M solution in diethyl ether. The reaction mixture was allowed to warm to 0°C, then was cooled to -20°C at which point it was quenched with 11.7 mL (151 mmol) of methyl chloroformate. The reaction mixture was allowed to warm to room temperature, then was partitioned between a saturated solution of sodium bicarbonate and ethyl acetate. The reaction was dried with sodium sulfate and the solvent evaporated. Flash chromatography yielded 18.39 g (96.2 mmol) of the yellow solid, 64%. Mass spectrum (APCI) m/e 192 p+1. $^1H$ NMR ($CDCl_3$, 400 MHz)_7.53(dd, J1 =7.1 Hz, 7.5 Hz, J = 8.2 Hz, 1H), 7.17(d, J=7.47 Hz, 1H), 6.78 (d, J = 7.8 Hz, 1H), 3.91 (s, 3H), 3.82(s, 3H) ppm.

C) Benzyl-methoxymethyl-trimethylsilanylmethyl-amine

To a 300 mL round bottom flask was charged 57.2 g (698 mmol) of 30 weight percent formaldehyde, which was cooled to 0°C. To the cold formaldehyde was added 89.92 g (485 mmol) of Benzyl-methoxymethyl-trimethylsilanylmethyl-amine. After 10 min, 60 mL of methanol were added drop-wise, followed by excessive potassium carbonate (105 g). The reaction mixture was stirred for 1 hour then the liquid phase was separated. More potassium carbonate was added to the liquid phase (20 g) and the reaction mixture was allowed to stir at room temperature for 72 hours. The reaction mixture was filtered and the excess methanol evaporated. The liquid was purified by vacuum distillation to yield

51.3 g (216 mmol) of clear liquid, 69%. Mass spectrum (APCI) m/e 238 p+1. $^1$H NMR ($CDCl_3$, 400 MHz)_7.34-7.30(m, 5H), 4.01(s, 2H), 3.77(s, 2H), 3.19(s, 3H), 2.19(s, 2H), 0.054(s, 9H) ppm.

D) 1-Benzyl-4-(6-methoxy-pyridin-2-yl)-2,5-dihydro-1H-pyrrole-3-carboxylic acid methyl ester

To a 15 mL round bottom flask was added 205 mg (1.07 mmol) of 3-(6-Methoxy-pyridin-2-yl)-prop-2-ynoic acid methyl ester, 305 mg (1.29 mmol) Benzyl-methoxymethyl-trimethylsilanylmethyl-amine and 5 mL of methylene chloride. The solution was cooled to 0°C. At 0°C. 107 microliters of 1M TFA in methylene chloride were added to the reaction mixture. The reaction mixture was allowed to stir at 0°C for 20 min, the ice bath was removed, and the solution was allowed to warm to room temperature. The solution stirred at room temperature for 2 hours, then the solvent was evaporated. Flash chromatography was performed with 20% ethyl acetate/hexanes to afford 295 mg (1.10 mmol) of the yellow oil, 85%. Mass spectrum (APCI) m/e 325 p+1. $^1$H NMR ($CDCl_3$, 400 MHz)_7.54(t, J = 8.3 Hz, 1H), 7.40-7.25(m, 6H) 6.66(d, J = 8.3 Hz, 1H), 4.08(t, 2H), 3.90(t, 2H), 3.86(s, 5H), 3.71 (s, 3H) ppm.

E) 1-Benzyl-4-(6-methoxy-pyridin-2-yl)-pyrrolidine-3-carboxylic acid methyl ester

To a 150 mL parr flask was charged 1.21g (3.75 mmol) of 1-Benzyl-4-(6-methoxy-pyridin-2-yl)-2,5-dihydro-1H-pyrrole-3-carboxylic acid methyl ester dissolved in 5 mL of methanol. To the flask was added 245 mg of 20 weight percent palladium hydroxide on activated carbon. The reaction mixture was placed on a Parr apparatus, the gas from the flask was purged and the reaction mixture subjected to 40 psi of hydrogen for 2 hours. Flash chromatography was performed with 20% to 50% EtOAc/Hexanes to afford 504 mg (0.647 mmol) of the yellow oil, 41%. Mass spectrum (APCI) m/e 327 p+1. $^1$H NMR ($CDCl_3$, 400 MHz)_7.40(dd, J1 = 7.5 Hz, J2 = 7.1 Hz, 1H), 7.33-7.19(m, 5H), 6.67(dd, J = 7.0 Hz, 1H), 3.84(s, 3H), 3.75-3.68(m, 3H), 3.42(dd, J = 2.1 Hz, J = 2.9 Hz, 1H), 3.26-3.19(m, 3H), 3.23(s, 3H), 3.00(t, J = 9.1 Hz, 1H), 2.70(t, J = 8.7 Hz, 1H) ppm.

F) [1-Benzyl-4-(6-methoxy-pyridin-2-yl]-pyrrolidin-3-yl]-methanol

To a 10 mL round bottom flask was added 203 mg (0.622 mmol) of 1-Benzyl-4-(6-methoxy-pyridin-2-yl)-pyrrolidine-3-carboxylic acid methyl ester and 3 mL of ethyl ether. The solution was cooled to 0°C treated with 996 microliters of 1 M lithium aluminum hydride in ethyl ether. The solution was stirred at 0°C for 1.5 hours, then was treated with a slurry of sodium sulfate decahydrate mixed 1:1 with diatomaceous earth in ether. The mixture was then filtered through diatomaceous earth and the solvent evaporated. The crude reaction mixture was chromatographed on silica gel eluting with 0.5% ammonium hydroxide/10% methanol/dichloromethane to afford 806 mg (0.240 mmol) of the yellow oil, [1-Benzyl-4-(6-methoxy-pyridin-2-yl)-pyrrolidin-3-yl]-methanol, 67%. Mass spectrum (APCI) m/e 299 p+1. $^1$H NMR ($CDCl_3$, 400 MHz)_7.5(dd, J = 7.47 Hz, J = 8.3 Hz, 1H), 7.38-7.21 (m, 6H), 6.90(d, J = 7.1, 1H), 6.59 (d, J= 8.3 Hz, 1H), 3.88(s, 3H), 3.77(s, 2H), 3.63-3.59(m, 1H), 3.42-3.24 (m, 2H), 3.05-2.97(m, 3H), 2.77-2.69(m, 2H) ppm.

G) 2-Benzyl-1,2,3,3a,8,8a-hexahvdro-2,7a-diaza-cyclopenta[a]inden-7-one

To a 35 mL round-bottom flask was added 597 mg (2.00 mmol) of [1-Benzyl-4-(6-methoxy-pyridin-2-yl)-pyrrolidin-3-yl]-methanol and 10 mL of dichloromethane. The mixture was cooled to 0°C and treated with 1.03g (8.00 mmol) N,N diisopropylethylamine, followed by the addition of 344 mg (3.00 mmol) of methansulfonyl chloride. The reaction mixture was stirred at 0°C for 1h, then allowed to warm to room temperature. The reaction mixture was stirred at room temperature for 16 hours then was partitioned between sodium bicarbonate and dichloromethane. The crude reaction mixture was dried with magnesium sulfate, and the solvent was evaporated. Flash chromatography was performed on silica gel using with 5-10% methanol/ethyl acetate to yield 40 mg (1.28 mmol) of the yellow oil, 64%. Mass spectrum (APCI) m/e 267 p+1. $^1$H NMR ($CDCl_3$, 400 MHz)_7.3(dd, 1H), 7.25-7.17(m, 5H), 6.35, d, J = 9.1 Hz, 1H), 6.00 (d, J = 7.1 Hz, 1H), 4.29(dd, J = 9.1 Hz, J = 13.3 Hz, 1H), 3.95(dd, 1H), 3.78(t, J = 7.9 Hz, 1H), 3.60(d, J = 12.9 Hz, 1H), 3.48 (d, J=12.9 Hz, 1H), 3.02(m, 1H), 3.54(d, J = 9.1 Hz, 1H), 2.66(t, 2H), 2.50(t, 1H) ppm.

H) 7-Oxo-3a,7,8,8a-tetrahydro-1H,3H-2,7a-diaza-cyclopenta[a]indene-2-carboxylic acid tert-butyl ester

To a 150 mL Parr flask was added 37.5 mg (0.141 mmol) of 2-Benzyl-1,2,3,3a,8,8a-hexahydro-2,7a-diaza-cyclopenta[a]inden-7-one dissolved in 3 mL of methanol. Added to the flask was 10 mg of 20 weight percent palladium hydroxide on activated carbon and 123 mg (0.563 mmol) of di-tert-butyl carbamate. The flask was purged of gas on a Parr apparatus, and the reaction mixture was subjected to 45 psi of hydrogen at 50°C for 2 hours. TLC taken at this point showed the reaction was not proceeding. The reaction mixture was filtered through diatomaceous earth, and the above amounts of palladium hydroxide on activated carbon and di-tert-butyl carbamate were added again. The reaction

mixture was subjected to the hydrogenation conditions above. The reaction was complete after 1 hour. The reaction mixture was filtered through diatomaceous earth and the solvent evaporated. Flash chromatography on silica gel eluting with 5% methanol/dichloromethane afforded a quantitative yield. Mass spectrum (APCI) m/e 277 p+1. $^1$H NMR ($CD_3OD$, 400 MHz)_7.55(dd, 1H), 6.42(dd, 2H), 4.20-4.11 (m, 2H), 4.00(t, 1H), 3.76-3.70(m, 3H), 3.31 (s, obsc), 3.04(dd, J = 6.6 Hz, J = 1.6 Hz, 1H) 1.40(s, 9H) ppm.

I) 1,2,3,3a,8,8a-Hexahydro-2,7a-diaza-cyclopenta[a]inden-7-one (hydrochloride salt)

To a 1 dram vial was added 18 mg (0.065 mmol) of 7-Oxo-3a,7,8,8a-tetrahydro-1H,3H-2,7a-diaza-cyclopenta [a]indene-2-carboxylic acid tert-butyl ester dissolved in 20% methanol/ethyl acetate. The reaction mixture was treated with 2 mL of 1 N HCl in methanol and stirred at room temperature for 16 hours. One additional mL of 1N HCl was added and the reaction mixture and the reaction mixture was heated to 50°C. After 4 hours the reaction was complete. The solvent was evaporated to afford a quantitative yield of 1,2,3,3a,8,8a-Hexahydro-2,7a-diaza-cyclopenta[a]inden-7-one (hydrochloride salt). Mass spectrum (APCI) m/e 177 p+1. $^1$H NMR ($CD_3OD$, 400 MHz)_7.80(dd, J = 7.5 Hz, J = 8.7 Hz, 1H), 7.81 (d, J = 7.5 Hz, 1H), 6.79(d, J = 7.1 Hz, 1H), 4.48-4.38(m, 2H), 4.28(dd, 1H), 3.73-3.51(m, 5H), 3.33(m, obsc., 1H) ppm.

**EXAMPLE 2**

A) 3-Benzyl-5-(6-methoxy-pyridin-2-yl)-3-aza-bicyclo[3,1,0]hexane-1-carboxylic acid methyl ester

Preparation of dimethylsulfoxonium ylide

To a 100 mL 3 necked round bottom flask was added 5.00 g (38.9 mmol) of trimethylsulfoxonium chloride and 38.9 mL of THF. The solution was placed under nitrogen, then 5.72 g (42.8 mmol) of 30 weight percent potassium hydride was added to the flask. The solution was heated under reflux for 4 hours then filtered. No rinses with THF were done to maintain an approximately 1 M stock solution of the ylide.

Preparation of 3-Benzyl-5-(6-methoxy-pyridin-2-yl)-3-aza-bicyclo[3.1.0]hexane-1-carboxylic acid methyl ester

To a 50 mL 3 necked round bottom flask under nitrogen was added 4.48 g (13.8 mmol) of 3-Benzyl-5-(6-methoxy-pyridin-2-yl)-3-aza-bicyclo[3.1.0]hexane-1-carboxylic acid methyl ester and 20 mL of the 1 M dimethylsulfoxonium ylide solution (above). The reaction mixture was allowed to stir at room temperature for 1.5 hours. The reaction mixture was treated with 20 mL of water, and then extracted with ethyl ether. The crude oil was dried with sodium sulfate and the solvent evaporated. Chromatography was done on flash 40-L eluting with 20% ethyl acetate/hexanes to afford 2.86 g (8.45 mmol) of 3-Benzyl-5-(6-methoxy-pyridin-2-yl)-3-aza-bicyclo[3.1.0]hexane-1-carboxylic acid methyl ester, 61%. Mass spectrum (APCI) m/e 339 p+1. $^1$H NMR ($CD_3OD$, 400 MHz)_7.52(dd, 1H), 7.31-7.20(m, 5H), 6.87(d, J = 7.5 Hz, 1H), 6.56(d, J = 7.9 Hz, 1H), 3.81 (s, 3H), 3.67(s, 2H), 3.38(s, 3H), 3.08-2.97(m, 4H), 1.94(d, J = 4.1 Hz, 1H), 1.89 (d, J = 4.1 Hz, 1H) ppm.

B) [3-Benzyl-5-(6-methoxy-pyridin-2-yl)-3-aza-bicyclo[3.1.0]hex-1-yl]-methanol

To a 10 mL round-bottom flask was added 296 mg (0.874 mmol) of 3-Benzyl-5-(6-methoxy-pyridin-2-yl)-3-aza-bicyclo[3.1.0]hexane-1-carboxylic acid methyl ester and 3 mL of ether. The solution was cooled to 0°C and was treated with 1.4 mL of 1 M lithium aluminum hydride in diethyl ether. The reaction mixture was treated with a slurry of 1:1 of sodium sulfate decahydrate:diatomaceous earth in ethyl ether. The crude reaction mixture was filtered through diatomaceous earth and the solvent evaporated. Flash chromatography was done on silica gel eluting with 0.5% ammonium hydroxide/5% methanol/dichloromethane afforded 258 mg (1.20 mmol) of the alcohol, 95%. Mass spectrum (AP) m/e 311 p+1. $^1$H NMR ($CDCl_3$, 400 MHz)_7.48(t, 1H), 7.30-7.19(m, 5H), 6.86(d, J = 0.83 Hz, 1H), 6.59(d, J = 0.83 Hz, 1H), 3.93(d, J = 12.4 Hz, 1H), 3.87(s, 3H), 3.67(d, J = 4.1, 2H), 3.42(d, J = 12.4 Hz, 1H), 3.17(d, J=8.3 Hz, 1H), 3.02(d, J = 8.7 Hz, 1H), 2.84(d, J = 8.7 Hz, 1H), 2.76(d, J = 8.3 Hz, 1H), 1.68(d, J=3.7 Hz, 1H), 1.07(d, J = 4.1 Hz, 1H) ppm.

C) 1-Hydroxymethyl-5-(6-methoxy-pyridin-2-yl)-3-aza-bicyclo[3.1.0]hexane-3-carboxylic acid tert-butyl ester:

To a 150 mL Parr flask, 59.0 mg (0.190 (mmol) of [3-Benzyl-5-(6-methoxy-pyridin-2-yl)-3-aza-bicyclo[3.1.0] hex-1-yl]-methanol dissolved in 10 mL of methanol was combined with 14 mg palladium hydroxide on activated carbon (20 wt %) and 166 mg (0.76 mmol) di-tert-butyl carbamate. The Parr flask was purged of gas on a Parr hydrogenation apparatus and the reaction mixture subjected to 45 psi of hydrogen at 50°C. The crude reaction mixture was filtered

through diatomaceous earth and the solvent evaporated. Flash chromatography was performed on silica gel eluting with 30% ethyl acetate/hexanes to afford 56.9 mg (0.178 mmol) of 1-Hydroxymethyl-5-(6-methoxy-pyridin-2-yl)-3-aza-bicyclo [3.1.0]hexane-3-carboxylic acid tert-butyl ester, 94%. Mass spectrum (AP) m/e 321 p+1. $^1H$ NMR ($CDCl_3$, 400 MHz)_ 7.5(t, 1H), 6.94(d, J = 0.83 Hz, 1H), 6.93(d, J = 0.83 Hz, 1H), 3.95-3.90(m, obsc.), 3.90(s, 3H), 3.81(d, J = 10.4 Hz, 2H), 3.68(d, J = 10.7 Hz, 2H), 1.43(s, 9H), 1.27(d, J = 5.0, 1H), 1.04(J = 5.0, 1H) ppm.

D) Cyclized BOC-Protected Cyclopronane:

To a 5 ml round bottom flask was added 53.9mg (0.168 mmol) of 1-Hydroxymethyl-5-(6-methoxy-pyridin-2-yl)-3-aza-bicyclo[3.1.0]hexane-3-carboxylic acid tert-butyl ester and 1.5 ml dichloromethane. The solution was cooled to 0°C and treated with 117 microliters (0.672 mmol) of N, N diisopropylethylamine followed by 19.6 microliters (0.253 mmol) of methanesulfonyl chloride. The reaction was stirred at 0°C for 1 h, then was allowed to warm to room temperature. The reaction stirred at room temperature for 16 hours then was partitioned between saturated sodium bicarbonate methylene chloride. The reaction mixture was dried with magnesium sulfate and the solvent evaporated. Chromatography -was performed on silica gel eluting with 2.5% MeOH/EtOAc to afford 39.8 mg (0.138 mmol) of the desired cyclized product, 82%. Mass spectrum (APCI) m/e 289 p+1. 7.51 (t, 1H), 6.35(t, 1H), 6.39(d, J = 8.7 Hz, 1H), 4.23(d, J = 6.6 Hz, 2H), 3.90-3.82(m, 2H), 3.65-3.53(m, 2H), 1.49(d, J = 5.8 Hz, 1H), 1.45(s, 9H), 1.32(d, J = 5.8 Hz, 1H) ppm.

E) Cyclized Cyclopropane (hydrochloride salt):

A one-dram vial was charged with 13 mg, 0.045 mmol of the substrate dissolved in a 1:1 mixture of ethyl acetate/hexanes. The reaction mixture was treated with 2 ml of 1 N HCl in methanol and stirred at room temperature for 16 hours. 1 additional mL of 1 N HCl in methanol was added to the reaction mixture and the solution was heated to 50°C. After four hours the reaction was complete and the solvent was evaporated to afford a quantitative yield. Mass spectrum (APCI) m/e 189 p+1. 7.75(t, 1H), 6.82(m, 1H), 6.66(d, J = 9.1, 1H), 4.41(d, J = 2.9 Hz, 2H), 3.81-3.63(m, 4H), 1.85(d, J = 7.1 Hz, 1H), 1.63(d, J = 7.1 Hz, 1H) ppm.

## Claims

1. A compound of formula I:

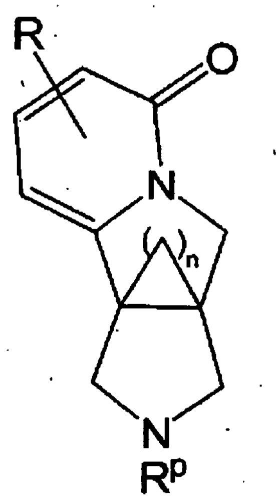

wherein:

$R^P$ is hydrogen, ($C_1$-$C_6$)alkyl, or benzyl;
R is hydrogen, ($C_1$-$C_6$)alkyl, ($C_2$-$C_6$)alkenyl, ($C_2$-$C_6$)alkynyl, hydroxy, amino, halo, cyano, aryl, wherein said aryl is selected from phenyl and naphthyl, heteroaryl, wherein said heteroaryl is selected from five to seven membered aromatic rings containing from one to four heteroatoms selected from oxygen, nitrogen and sulfur, -$SO_q$($C_1$-$C_6$)alkyl or -$SO_q$($C_1$-$C_6$)aryl wherein q is zero, one or two, ($C_1$-$C_8$)alkylamino-, [($C_1$-$C_6$)alkyl]$_2$amino-,

$-CO_2R^1$, $-CONR^2R^3$, $-SO_2NR^4R^5$, $-C(=O)R^6$, $-XC(=O)R^6$ wherein X is $(C_1-C_6)$alkylene, aryl-$(C_0-C_3)$alkyl- or aryl-$(C_0-C_3)$alkyl-O-, heteroaryl-$(C_0-C_3)$alkyl- or heteroaryl-$(C_0-C_3)$alkyl-O-, arid $X^2(C_0-C_6)$alkoxy-$(C_0-C_6)$alkyl-, wherein $X^2$ is absent or $X^2$ is $(C_1-C_6)$alkylamino- or $[(C_1-C_6)alkyl]_2$amino-, and wherein the $(C_0-C_6)$alkoxy-$(C_0-C_6)$alkyl- moiety of said $X^2(C_0-C_6)$alkoxy-$(C_0-C_6)$alkyl- contains at least one carbon atom, and wherein from one to three of the carbon atoms of said $(C_0-C_6)$alkoxy-$(C_0-C_6)$alkyl- moiety may optionally be replaced by an oxygen, nitrogen or sulfur atom, with the proviso that any two such heteroatoms must be separated by at least two carbon atoms, and wherein any of the alkyl moieties of said $(C_0-C_{6)}$alkoxy-$(C_0-C_6)$alkyl- may be optionally substituted with from two to seven fluorine atoms, and wherein one of the carbon atoms of each of the alkyl moieties of said aryl-$(C_0-C_3)$alkyl- and said heteroaryl-$(C_0-C_3)$alkyl- may optionally be replaced by an oxygen, nitrogen or sulfur atom, and wherein each of the foregoing alkenyl, alkynyl, aryl and heteroaryl groups may optionally be substituted with one or more substituents independently selected from $(C_1-C_6)$alkyl optionally substituted with from one to seven fluorine atoms, $(C_1-C_6)$alkoxy optionally substituted with from two to seven fluorine atoms, halo, $(C_2-C_6)$alkenyl, $(C_2-C_6)$alkynyl, hydroxy, nitro, cyano, amino, $(C_1-C_6)$alkylamino-, $[(C_1-C_6)alkyl]_2$amino-, $-CO_2R^1$, $-CONR^2R^3$, $-SO_2NR^4R^6$, $-C(=O)R^6$ and $-XC(=O)R^6$;
each $R^1$, $R^2$, $R^3$, $R^4$, $R^5$ and $R^6$ is selected, independently, from hydrogen and $(C_1-C_6)$ alkyl, or $R^2$ and $R^3$, or $R^4$ and $R^5$ together with the nitrogen to which they are attached, form a pyrrolidine, piperidine, morpholine, azetidine, piperazine, -N-$(C_1-C_6)$alkylpiperazine or thiomorpholine ring, or a thiomorpholine ring wherein the ring sulfur is replaced with a sulfoxide or sulfone;
each X is, independently, $(C_1-C_6)$alkylene; and
n is an integer from zero to 2;
o, a pharmaceutically acceptable salt of said compound.

**2.** A compound according to claim 1 wherein $R^P$ is hydrogen.

**3.** A compound according to claim 2 wherein R is hydrogen. $(C_1-C_6)$alkyl, $(C_2-C_6)$alkenyl, $(C_2-C_6)$alkynyl, hydroxy, amino, halo, cyano, phenyl, naphthyl, thienyl, pyridyl, pyrimidyl, pyridazyl, oxazolyl, isoxazolyl, thiazolyl or isothiazolyl.

**4.** A compound according to claim 1 selected from the group consisting of:

2-Benzyl-1,2,3,3a,8,8a-hexahydro-2,7a-diaza-cyclopenta[a]inden-7-one;
1,2,3,3a,8,8a-Hexahydro-2,7a-diaza-cyclopenta[a]inden-7-one;
2,3-Dihydro-1H,6H-3a,9b-methapyrrolo[3,4-a]indolizine-6-one;
2-Benzyl-2,3-dihydro-1H,6H-3a,9b-methapymolo[3,4-a]indolizine-6-one; and,
t-Butyl 6-oxo-2,3-dihydro-1H,6H-3a,9b-methapyrrolo[3,4-a]indolizine-2-carboxylate.

**5.** A compound of formula I wherein n, R, $R^1$, $R^2$, $R^3$, $R^4$, $R^5$, $R^6$, X and $X^2$ are as defined in claim 1 and $R^P$ is a protective group selected from the group consisting of t-butoxycarbonyl, trifluoroacetyl, CBz, FMOC, Bz, methyl and acetyl.

**6.** A pharmaceutical composition comprising an effective amount of a compound according to any one of claims 1 to 4, or a pharmaceutically acceptable saft thereof, and a pharmaceutically acceptable carrier.

**7.** A compound according to any one of claims 1 to 4. or a pharmaceutically acceptable salt thereof, for use as a medicament.

**8.** Use of a compound according to any one of claims 1 to 4, or a pharmaceutically acceptable salt thereof, in the manufacture of a medicament for reducing nicotine addiction or aiding in the cessation or lessening of tobacco use in a mammal.

**9.** Use of a compound according to any one of claims 1 to 4, or a pharmaceutically acceptable salt thereof, in the manufacture of a medicament for treating an addictive disorder or neurological or mental disorder related to a decrease in cholinergic function in a mammal.

**10.** Use of a compound according to any one of claims 1 to 4. or a pharmaceutically acceptable salt thereof, in the manufacture of a medicament for treating a disorder or condition selected from Huntington's Chorea, tardive dyskinesia, hyperkinesia, mania, dyslexia, schizophrenia, analgesia, attention deficit disorder (ADD), mufti-infarct dementia, age related cognitive decline, epilepsy, senile dementia of the Alzheimer's type, Parkinson's disease, (PD)

attention deficit hyperactivity disorder (ADHD), anxiety, obesity, Tourette's syndrome and ulcerative colitis, in a mammal.

**11.** A compound according to any one of claims 1 to 4, or a pharmaceutically acceptable salt thereof, for use in reducing nicotine addiction or aiding in the cessation or lessening of tobacco use in a mammal.

**12.** A compound according to any one of claims 1 to 4, or a pharmaceutically acceptable salt thereof, for use in treating an addictive disorder or neurological or mental disorder related to a decrease in cholinergic function in a mammal.

**13.** A compound according to any one of claims 1 to 4, or a pharmaceutically acceptable salt thereof, for use in treating a disorder or condition selected from Huntington's Chorea, tardive dyskinesia, hyperkinesia, mania, dyslexia, schizophrenia, analgesia, attention deficit disorder (ADD), multi-infarct dementia, age related cognitive decline, epilepsy, senile dementia of the Alzheimer's type, Parkinson's disease, (PD) attention deficit hyperactivity disorder (ADHD), anxiety, obesity, Tourette's syndrome and ulcerative colitis, in a mammal.

**Patentansprüche**

**1.** Verbindung der Formel I

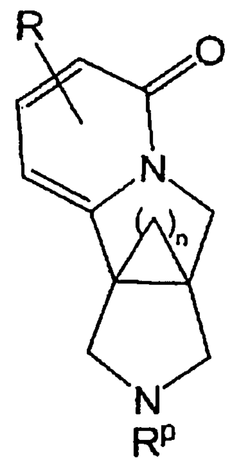

worin:

$R^P$ Wasserstoff, $(C_1-C_6)$Alkyl oder Benzyl ist;
R ist Wasserstoff, $(C_1-C_6)$Alkyl, $(C_2-C_6)$Alkenyl, $(C_2-C_6)$Alkinyl, Hydroxy, Amino, Halogen, Cyano, Aryl, wobei das Aryl ausgewählt ist aus Phenyl und Naphthyl, Heteroaryl, wobei das Heteroaryl ausgewählt ist aus 5- bis 7-gliedrigen aromatischen Ringen, enthaltend 1 bis 4 Heteroatome, ausgewählt aus Sauerstoff, Stickstoff und Schwefel, $-SO_q(C_1-C_6)$Alkyl oder $-SO_q(C_1-C_6)$Aryl, wobei q 0, 1 oder 2 ist, $(C_1-C_6)$Alkylamino-, $[(C_1-C_6)$Alkyl$]_2$amino-, $-CO_2R^1$ $-CONR^2R^3$, $-SO_2NR^4R^5$, $-C(=O)R^6$, $-XC(=O)R^6$, wobei X ist $(C_1-C_6)$Alkylen, Aryl-$(C_0-C_3)$alkyl- oder Aryl-$(C_0-C_3)$alkyl-O-, Heteroaryl-$(C_0-C_3)$alkyl- oder Heteroaryl-$(C_0-C_3)$alkyl-O-, und $X^2(C_0-C_6)$Alkoxy-$(C_0-C_6)$alkyl-, wobei $X^2$ nicht vorhanden ist oder $X^2$ $(C_1-C_6)$Alkylamino- oder $[(C_1-C_6)$Alkyl$]_2$amino- ist und wobei die $(C_0-C_6)$Alkoxy-$(C_0-C_6)$alkyl-Gruppierung von $X^2(C_0-C_6)$ Alkoxy- $(C_0-C_6)$alkyl- mindestens ein Kohlenstoffatom enthält und wobei von 1 bis 3 der Kohlenstoffatome der $(C_0-C_6)$Alkoxy-$(C_0-C_6)$alkyl-Gruppierung gegebenenfalls durch ein Sauerstoff-, Stickstoff- oder Schwefelatom ersetzt sein können, mit der Maßgabe, dass jegliche zwei derartige Heteroatome durch mindestens zwei Kohlenstoffatome getrennt sein müssen, und wobei jede der Alkylgruppierungen von $(C_0-C_6)$Alkoxy-$(C_0-C_6)$alkylgegebenenfalls mit 2 bis 7 Fluoratomen substituiert sein kann, und wobei eines der Kohlenstoffatome jeder dieser Alkylgruppierungen von Aryl-$(C_0-C_3)$alkyl- und von Heteroaryl-$(C_0-C_3)$alkyl- gegebenenfalls durch ein Sauerstoff-, Stickstoff-, oder Schwefelatom ersetzt sein kann, und wobei jede der vorangegangenen Alkenyl-, Alkinyl-, Aryl- und Heteroarylgruppen gegebenenfalls mit einem oder mehreren Substituenten substituiert sein kann, unabhängig ausgewählt aus $(C_1-C_6)$Alkyl, gegebenenfalls substituiert mit 1 bis 7 Fluoratomen, $(C_1-C_6)$Alkoxy, gegebenenfalls substituiert mit 2 bis

7 Fluoratomen, Halogen, $(C_2-C_6)$Alkenyl, $(C_2-C_6)$Alkinyl, Hydroxy, Nitro, Cyano, Amino, $(C_1-C_6)$Alkylamino-, $[(C_1-C_6)Alkyl]_2$amino-, $-CO_2R^1$, $-CONR^2R^3$, $-SO_2NR^4R^5$, $-C(=O)R^6$ und $-XC(=O)R^6$;
jedes $R^1$, $R^2$, $R^3$, $R^4$, $R^5$ und $R^6$ unabhängig ausgewählt ist aus Wasserstoff und $(C_1-C_6)$Alkyl, oder $R^2$ und $R^3$ oder $R^4$ und $R^5$ zusammen mit dem Stickstoff, an den sie gebunden sind, einen Pyrrolidin-, Piperidin-, Morpholin-, Azetidin-, Piperazin-, $-N-(C_1-C_6)$Alkylpiperazin- oder Thiomorpholinring bilden oder einen Thiomorpholinring, wobei der Ringschwefel durch ein Sulfoxid oder Sulfon ersetzt ist;
jedes X unabhängig $(C_1-C_6)$Alkylen ist; und
n eine ganze Zahl von 0 bis 2 ist;
oder ein pharmazeutisch verträgliches Salz der Verbindung.

**2.** Verbindung gemäß Anspruch 1, worin $R^P$ Wasserstoff ist.

**3.** Verbindung gemäß Anspruch 2, worin R Wasserstoff, $(C_1-C_6)$Alkyl, $(C_2-C_6)$Alkenyl, $(C_2-C_6)$Alkinyl, Hydroxy, Amino, Halogen, Cyano, Phenyl, Naphthyl, Thienyl, Pyridyl, Pyrimidyl, Pyridazyl, Oxazolyl, Isoxazolyl, Thiazolyl oder Isothiazolyl ist.

**4.** Verbindung gemäß Anspruch 1, ausgewählt aus der Gruppe bestehend aus:

2-Benzyl-1,2,3,3a,8,8a-hexahydro-2,7a-diazacyclopenta[a]inden-7-on;
1,2,3,3a,8,8a-Hexahydro-2,7a-diazacyclopenta[a]inden-7-on;
2,3-Dihydro-1H,6H-3a,9b-methapyrrolo[3,4-a]indolizin-6-on;
2-Benzyl-2,3-dihydro-1H,6H-3a,9b-methapyrrolo[3,4-a]indolizin-6-on; und
t-Butyl-6-oxo-2,3-dihydro-1H,6H-3a,9b-methapyrrolo[3,4-a]indolizin-2-carboxylat.

**5.** Verbindung der Formel I, worin n, R, $R^1$, $R^2$, $R^3$, $R^4$, $R^5$, $R^6$, X und $X^2$ wie in Anspruch 1 definiert sind und $R^P$ eine Schutzgruppe, ausgewählt aus der Gruppe, bestehend aus t-Butoxycarbonyl, Trifluoracetyl, CBz, FMOC, Bz, Methyl und Acetyl, ist.

**6.** Pharmazeutische Zusammensetzung, umfassend eine wirksame Menge einer Verbindung gemäß einem der Ansprüche 1 bis 4 oder eines pharmazeutisch verträglichen Salzes davon und einen pharmazeutisch verträglichen Träger.

**7.** Verbindung gemäß einem der Ansprüche 1 bis 4 oder ein pharmazeutisch verträgliches Salz davon zur Verwendung als Medikament.

**8.** Verwendung einer Verbindung gemäß einem der Ansprüche 1 bis 4 oder eines pharmazeutisch verträglichen Salzes davon zur Herstellung eines Medikaments zum Verringern von Nikotinabhängigkeit oder zum Unterstützen beim Aufhören oder Verringern des Verbrauchs von Tabak bei einem Säuger.

**9.** Verwendung einer Verbindung gemäß einem der Ansprüche 1 bis 4 oder eines pharmazeutisch verträglichen Salzes davon bei der Herstellung eines Medikaments zum Behandeln einer Abhängigkeitsstörung oder einer neurologischen oder mentalen Störung mit Bezug zu einer Abnahme bei der cholinergen Funktion bei einem Säuger.

**10.** Verwendung einer Verbindung gemäß einem der Ansprüche 1 bis 4 oder eines pharmazeutisch verträglichen Salzes davon bei der Herstellung eines Medikaments zum Behandeln einer Störung oder eines Zustands, ausgewählt aus Huntington-Chorea, Spätdyskinesie, Hyperkinesie, Manie, Dyslexie, Schizophrenie, Analgesie, Aufmerksamkeitsschwäche-Störung (ADD), Multiinfarktdemenz, altersbedingter Wahrnehmungs- bzw. Bewusstseinsabnahme, Epilepsie, seniler Demenz des Alzheimer-Typs, Parkinsonscher Krankheit (PD), Aufmerksamkeits- und Hyperaktivitätsstörung (ADHD), Angst, Adipositas, Tourette-Syndrom und Colitis ulcerosa, bei einem Säuger.

**11.** Verbindung gemäß einem der Ansprüche 1 bis 4 oder ein pharmazeutisch verträgliches Salz davon zur Verwendung beim Verringern von Nikotinabhängigkeit oder zum Unterstützen beim Aufhören oder Verringern des Verbrauchs von Tabak bei einem Säuger.

**12.** Verbindung gemäß einem der Ansprüche 1 bis 4 oder ein pharmazeutisch verträgliches Salz davon zur Verwendung beim Behandeln einer Abhängigkeitsstörung oder einer neurologischen oder mentalen Störung mit Bezug zu einer Abnahme bei der cholinergen Funktion bei einem Säuger.

13. Verbindung gemäß einem der Ansprüche 1 bis 4 oder ein pharmazeutisch verträgliches Salz davon zur Verwendung beim Behandeln einer Störung oder eines Zustands, ausgewählt aus Huntington-Chorea, Spätdyskinesie, Hyperkinesie, Manie, Dyslexie, Schizophrenie, Analgesie, Aufmerksamkeitsschwäche-Störung (ADD), Multiinfarktdemenz, altersbedingter Wahrnehmungs- bzw. Bewusstseinsabnahme, Epilepsie, seniler Demenz des Alzheimer-Typs, Parkinsonscher Krankheit (PD), Aufmerksamkeits- und Hyperaktivitätsstörung (ADHD), Angst, Adipositas, Tourette-Syndrom und Colitis ulcerosa, bei einem Säuger.

## Revendications

1. Composé de formule I :

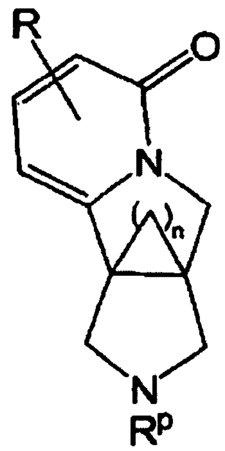

dans laquelle :

$R^p$ représente l'hydrogène, alkyle en $C_1$-$C_6$ ou benzyle ;
R représente l'hydrogène, alkyle en $C_1$-$C_6$, alkényle en $C_2$-$C_6$, alkynyle en $C_2$-$C_6$, hydroxy, amino, halogéno, cyano, aryle, ledit groupe aryle étant sélectionné parmi phényle, naphtyle et hétéroaryle, ledit groupe hétéroaryle étant sélectionné parmi des noyaux aromatiques ayant de cinq à sept éléments et contenant de un à quatre hétéroatomes sélectionnés parmi l'oxygène, l'azote et le soufre, $-SO_q$(alkyle en $C_1$-$C_6$) ou $-SO_q$(aryle en $C_1$-$C_6$), où q représente zéro, un ou deux, (alkyle en $C_1$-$C_6$) amino-, (alkyle en $C_1$-$C_6$)$_2$amino-, $-CO_2R^1$, $-CONR^2R^3$, $-SO_2NR^4R^5$, $-C(=O)R^6$, $-XC(=O)R^6$, où X représente alkylène en $C_1$-$C_6$, aryl-(alkyle en $C_0$-$C_3$)- ou aryl-(alkyle en $C_0$-$C_3$)-O-, hétéroaryl- (alkyle en $C_0$-$C_3$)- ou hétéroaryl- (alkyle en $C_0$-$C_3$)-O-, et $X^2$ (alcoxy en $C_0$-$C_6$)-(alkyle en $C_0$-$C_6$)-, où $X^2$ est absent ou $X^2$ représente (alkyle en $C_1$-$C_6$) amino- ou (alkyle en $C_1$-$C_6$)$_2$amino-, et où le groupe fonctionnel (alcoxy en $C_0$-$C_6$)-(alkyle en $C_0$-$C_6$)- dudit groupe $X^2$ (alcoxy en $C_0$-$C_6$)-(alkyle en $C_1$-$C_6$)- contient au moins un atome de carbone, un à trois atomes de carbone dudit groupe fonctionnel (alcoxy en $C_0$-$C_6$)-(alkyle en $C_0$-$C_6$)- pouvant être facultativement remplacé(s) par un atome d'oxygène, d'azote ou de soufre, étant entendu que les deux hétéroatomes de toute paire de ces hétéroatomes doivent être séparés par au moins deux atomes de carbone, et chacun des groupes fonctionnels alkyle dudit groupe (alcoxy en $C_0$-$C_6$)-(alkyle en $C_0$-$C_6$)- pouvant être facultativement substitué par de deux à sept atomes de fluor, l'un des atomes de carbone de chacun des groupes fonctionnels alkyle dudit groupe aryl-(alkyle en $C_0$-$C_3$)- et dudit groupe hétéroaryl-(alkyle en $C_0$-$C_3$)- pouvant être facultativement remplacé par un atome d'oxygène, d'azote ou de soufre, chacun des groupes alkényle, alkynyle, aryle et hétéroaryle mentionnés ci-dessus pouvant être facultativement substitué par un ou plusieurs substituants indépendamment sélectionné(s) parmi alkyle en $C_1$-$C_6$ facultativement substitué par de un à sept atomes de fluor, alcoxy en $C_1$-$C_6$ facultativement substitué par de deux à sept atomes de fluor, halogéno, alkényle en $C_2$-$C_6$, alkynyle en $C_2$-$C_6$, hydroxy, nitro, cyano, amino, (alkyle en $C_1$-$C_6$) amino-, (alkyle en $C_1$-$C_6$)$_2$amino-, $-CO_2R^1$, $-CONR^2R^3$, $-SO_2NR^4R^5$, $-C(=O)R^6$ et $-XC(=O)R^6$ ;
chaque $R^1$, $R^2$, $R^3$, $R^4$ $R^5$ et $R^6$ étant sélectionné, indépendamment, parmi l'hydrogène et alkyle en $C_1$-$C_6$, ou $R^2$ et $R^3$, ou $R^4$ et $R^5$, ensemble avec l'atome d'azote auquel ils sont liés, forment un noyau pyrrolidine, pipéridine, morpholine, azétidine, pipérazine, -N-(alkyle en $C_1$-$C_6$)pipérazine ou thiomorpholine, ou un noyau thiomorpholine dans lequel l'atome de soufre du noyau est remplacé par un sulfoxyde ou une sulfone ;

chaque X représentant, indépendamment, alkylène en $C_1$-$C_6$ ; et
n étant un entier entre zéro et 2 ;

ou sel pharmaceutiquement acceptable dudit composé.

**2.** Composé selon la revendication 1, dans lequel $R^p$ représente l'hydrogène.

**3.** Composé selon la revendication 2, dans lequel R représente l'hydrogène, alkyle en $C_1$-$C_6$, alkényle en $C_2$-$C_6$, alkynyle en $C_2$-$C_6$, hydroxy, amino, halogéno, cyano, phényle, naphtyle, thiényle, pyridyle, pyrimidyle, pyridazyle, oxazolyle, isoxazolyle, thiazolyle ou isothiazolyle.

**4.** Composé selon la revendication 1, sélectionné dans le groupe consistant en :

- la 2-benzyl-1,2,3,3a,8,8a-hexahydro-2,7a-diaza-cyclopenta[a] indén-7-one ;
- la 1,2,3,3a,8,8a-hexahydro-2,7a-diaza-cyclopenta[a]indén-7-one ;
- la 2,3-dihydro-1H,6H-3a,9b-métapyrrolo[3,4-a]indolizin-6-one ;
- la 2-benzyl-2,3-dihydro-1H,6H-3a,9b-métapyrrolo[3,4-a] indolizin-6-one ; et
- le 6-oxo-2,3-dihydro-1H,6H-3a,9b-métapyrrolo[3,4-a]indolizine-2-carboxylate de t-butyle.

**5.** Composé de formule I dans lequel n, R, $R^1$, $R^2$, $R^3$, $R^4$, $R^5$, $R^6$, X et $X^2$ sont tels que définis dans la revendication 1 et $R^p$ est un groupe protecteur sélectionné dans le groupe consistant en t-butoxycarbonyle, trifluoroacétyle, CBz, FMOC, Bz, méthyle et acétyle.

**6.** Composition pharmaceutique comprenant une quantité efficace d'un composé selon l'une quelconque des revendications 1 à 4, ou d'un sel pharmaceutiquement acceptable correspondant, et un support pharmaceutiquement acceptable.

**7.** Composé selon l'une quelconque des revendications 1 à 4, ou sel pharmaceutiquement acceptable correspondant, destiné à une utilisation en tant que médicament.

**8.** Utilisation d'un composé selon l'une quelconque des revendications 1 à 4, ou d'un sel pharmaceutiquement acceptable correspondant, dans la fabrication d'un médicament réduisant l'addiction à la nicotine ou aidant à l'arrêt ou à la diminution de l'utilisation du tabac chez un mammifère.

**9.** Utilisation d'un composé selon l'une quelconque des revendications 1 à 4, ou d'un sel pharmaceutiquement acceptable correspondant, dans la fabrication d'un médicament pour traiter un trouble addictif ou un trouble neurologique ou mental lié à une diminution de la fonction cholinergique chez un mammifère.

**10.** Utilisation d'un composé selon l'une quelconque des revendications 1 à 4, ou d'un sel pharmaceutiquement acceptable correspondant, dans la fabrication d'un médicament pour traiter un trouble ou un état sélectionné parmi la chorée de Huntington, la dyskinésie tardive, l'hyperkinésie, la manie, la dyslexie, la schizophrénie, l'analgésie, le trouble du déficit d'attention (ADD), la démence multi-infarctus, le déclin cognitif lié à l'âge, l'épilepsie, la démence sénile de type Alzheimer, la maladie de Parkinson (PD), le trouble de déficit d'attention avec hyperactivité (ADHD), l'anxiété, l'obésité, le syndrome de Tourette et la colite ulcérante, chez un mammifère.

**11.** Composé selon l'une quelconque des revendications 1 à 4, ou sel pharmaceutiquement acceptable correspondant, destiné à une utilisation pour réduire l'addiction à la nicotine ou aider à l'arrêt ou à la diminution de l'utilisation du tabac chez un mammifère.

**12.** Composé selon l'une quelconque des revendications 1 à 4, ou sel pharmaceutiquement acceptable correspondant, destiné à une utilisation pour traiter un trouble addictif ou un trouble neurologique ou mental lié à une diminution de la fonction cholinergique chez un mammifère.

**13.** Composé selon l'une quelconque des revendications 1 à 4, ou sel pharmaceutiquement acceptable correspondant, destiné à une utilisation pour traiter un trouble ou un état sélectionné parmi la chorée de Huntington, la dyskinésie tardive, l'hyperkinésie, la manie, la dyslexie, la schizophrénie, l'analgésie, le trouble du déficit d'attention (ADD), la démence multi-infarctus, le déclin cognitif lié à l'âge, l'épilepsie, la démence sénile de type Alzheimer, la maladie de Parkinson (PD), le trouble de déficit d'attention avec hyperactivité (ADHD), l'anxiété, l'obésité, le syndrome de

Tourette et la colite ulcérante, chez un mammifère.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 08963852 B
- US 60070245 B


### Non-patent literature cited in the description


- **PAUL RYLANDER.** Catalytic Hydrogenation in Organic Synthesis. Academic Press Inc, 1979, 31-63
- **LO, P. et al.** *Org. Lett.,* 2001, vol. 3, 2819
- **CRIMMINS, M.T. et al.** *Tetrahedron Lett,* 1994, vol. 35, 1657-60
- **HERZOG. H. et al.** *Tetrahedron,* 1986, vol. 42, 3547-58
- **TOBE, Y. et al.** *Tetrahedron Left.,* 1986, vol. 27, 2905-06
- **LIPPIELLO, P. M. ; FERNANDES, K. G.** The Binding of L-[3H]Nicotine To A Single Class of High-Affinity Sites in Rat Brain Membranes. *Molecular Pharm.,* 1986, vol. 29, 448-54
- **ANDERSON, D. J. ; ARNERIC, S. P.** Nicotinic Receptor Binding of H-Cystisine, H-Nicotine and H-Methylcarmbamylcholine In Rat Brain. *European J. Pharm.,* 1994, vol. 253, 261-67
- **LIPPIELLO ; FERNANDEZ.** *Molec Pharmacol,* 1986, vol. 29, 448-454